# EUROPEAN PATENT APPLICATION

(11) **EP 4 088 646 A1**
(43) Date of publication of application: **16.11.2022**
(21) Application number: 20912206.8
(22) Date of filing: 30.12.2020
(51) Int. Cl.: A61B 1/32

(54) **VISUAL DIAGNOSIS AND TREATMENT SYSTEM WITH CAVITY DILATATION DEVICE**

(30) Priority: 08.01.2020 CN 202010019834
(71) Applicant: Zhou, Xing, Guangzhou, Guangdong 510663 (CN)
(72) Inventor: Zhou, Xing, Guangzhou, Guangdong 510663 (CN)
(74) Representative: Boult Wade Tennant LLP
(86) International application number: PCT/CN2020/141172
(87) International publication number: WO 2021/139575

(57) **Abstract**

A visualized medical system with a cavity expanding device includes a cavity expanding mechanism and an observation mechanism. The cavity expanding mechanism includes an expanding end arranged at a front end of the operating mechanism and configured to expand a cavity at the front end of the observation mechanism to expand a field of view of the observation mechanism. The observation mechanism includes a lighting module, a lens module, a signal processing module, and a housing. An image and a video observed in the lens module in a light field of the lighting module are processed by the signal processing module, and then outputted to a display system by a circuit system for displaying. The visualized medical system with a cavity expanding device can introduce a surgical instrument into a body from a sheath to perform various surgical operations such as biopsy sampling, electrocution, electrocoagulation, and ablation during observation.

## Description

### FIELD OF THE INVENTION

The present invention relates to an endoscopic surgical instrument, and in particular, to a visualized medical system with a cavity expanding device applicable to an endoscopic surgery.

### BACKGROUND OF THE INVENTION

As endoscopic technologies develop, many examinations and surgical processes can be completed endoscopically, including gastroscopy, colonoscopy, hysteroscopy, biopsy, abortion, and the like.

Conventionally, various mirrors, such as a laparoscope, a thoracoscope, or a hysteroscope is inserted into a human body, and an examination or a surgery is performed under direct observation with a lens. In order to ensure a small surgical trauma, a size of a surgical incision is usually minimized. Therefore, diameters of matched mirrors and surgical instruments are relatively small.

However, tissues and organs in human bodies are soft. Therefore, without the action of an external force, cavities of the tissues and the organs, such as a uterus, a stomach, and intestines are usually closed. Due to collapse of tissues in front of an endoscope, especially the cavities such as the uterus and the intestines, an observation area in the front is very small when a small-diameter endoscopic instrument enters the cavities. Therefore, a clinical observation effect is usually undesirable.

Thus, it is necessary to further improve existing endoscopic devices to realize a more desirable clinical observation effect. In addition, in the prior art, observation and surgical processes are usually separated. In a clinical process, different instruments are required to be changed for operation, especially for biopsy sampling, laser ablation, and the like. This is very inconvenient. Therefore, it is necessary to further improve and integrate functions of the existing endoscopic devices.

### SUMMARY

According to a visualized medical system with a cavity expanding device of the present invention, a front end of an observation system can be expanded by an expanding end of an expanding mechanism arranged at the front end of the observation system, so as to effectively expand a field of view. In this way, a clinical effect is very desirable.

A visualized medical system with a cavity expanding device of the present invention is provided. The visualized medical system 100 with a cavity expanding device includes a cavity expanding mechanism 1, an observation mechanism 2, a circuit system 3, a power supply system 104, an operating mechanism 5, and a display system 102, where
the cavity expanding mechanism 1 includes an expanding end 11, and the expanding end 11 is arranged at a front end 5-1 of the operating mechanism 5, and is configured to expand a cavity in front of the observation mechanism 2 to expand a field of view of the observation mechanism 2;
the observation mechanism 2 includes a lighting module 21, a lens module 22, a signal processing module 23, and a housing 24, where an image and a video observed in the lens module 22 in a light field of the lighting module 21 are processed by the signal processing module 23, and then outputted to the display system 102 by the circuit system 3 for displaying, and the lighting module 21, the lens module 22, and the circuit system 3 are connected and then mounted in the housing 24 in a sealed manner; and
the observation mechanism 2 is connected to the display system 102 by the circuit system 3 and the power supply system 104.

According to the visualized medical system with a cavity expanding device of the present invention, the expanding end 11 of the cavity expanding mechanism 1 is arranged at a front end of a camera 22-1 of the observation mechanism 2, and the expanding end 11 can expand the cavity in front of the observation mechanism 2 to expand a field of view of the observation mechanism 2. This is especially suitable for examination and surgery processes such as hysteroscopy or colonoscopy. During use, the visualized medical system with a cavity expanding device of the present invention is inserted into the cavity, the expanding end 11 expands the cavity, the observation mechanism 2 observes the expanded area, and image and video signals observed in the lens module 22 are outputted to the display system 102 by the circuit system 3 for displaying. Since the cavity in front of the observation mechanism 2 is expanded, a field of view of the lens module 22 is larger. Therefore, a clinical observation effect is more desirable.

The observation mechanism 2 and the cavity expanding mechanism 1 may be arranged separately or as a whole.

In order to reduce clinical costs, the observation mechanism 2 and the cavity expanding mechanism 1 may be arranged separately. A sheath 12 is arranged on the cavity expanding mechanism 1, and the observation mechanism 2 is sealed. During clinical observation, the observation mechanism 2 is placed in a channel 12-3 of the sheath 12. After observation is completed, the observation mechanism 2 is removed. The observation mechanism 2 can be reused after disinfection and sterilization, and only the cavity expanding mechanism 1 is used once. In this way, the clinical costs can be significantly reduced.

When the observation mechanism 2 and the cavity expanding mechanism 1 are arranged as a whole, the sheath of the cavity expanding mechanism 1 constitutes the housing 24. The lighting module 21 and the lens module 22 of the observation mechanism 2 and the circuit system 3 are sealed in the sheath 12.

The expanding end 11 is an expanding mesh 11-1. The camera 22-1 may directly observe a situation behind the expanding mesh 11-1 through mesh gaps. Therefore, even if the expanding mesh 11-1 is not made of a transparent material, full observation of a surgical process and panoramic observation of a surgical area can be realized.

A camera 22-1 of the lens module 22 is arranged at a rear end of the expanding mesh 11-1, and the expanding mesh 11-1 is located within a field of view of the camera 22-1.

The technical solution of arranging the camera 22-1 at the rear end can ensure that the expanding mesh 11-1 is completely within the field of view of the camera 22-1. In addition, since an observation angle of the camera 22-1 is oriented toward a front of a surgical or examination area, there is no observation blind in the above process. Therefore, the technical solution of arranging the camera at the rear end causes processes of clinical examination and surgery to be safer and more effective.

A camera 22-1 of the lens module 22 is arranged inside the expanding mesh 11-1. Since the camera 22-1 is arranged inside the expanding mesh 11-1, surrounding tissues and organs can be observed three-dimensionally by adjusting an orientation of the camera 22-1 or arranging a plurality of cameras 22-1 spatially distributed.

An observation direction of a camera 22-1 of the lens module 22 is adjustable. By adjusting the observation direction of the camera 22-1, the surrounding tissues can be observed in all directions. In this way, the clinical observation effect is more comprehensive.

An adjustment mechanism 22-11 of the camera 22-1 configured to adjust the observation direction is arranged on a handle 52 of the operating mechanism 5. Since the adjustment mechanism 22-11 is arranged on the handle 52, the observation direction of the camera 22-1 can be adjusted very conveniently outside a body during the clinical use. Therefore, the operation is simpler.

An orientation of a working portion of the expanding end 11 is adjustable. The orientation of the working portion of the expanding end 11 may be adjusted as required, so that the expanding end can adapt to a structure and a shape of a surrounding cavity more effectively, and a more desirable clinical use effect can be achieved, especially for areas that are difficult to remove or observe such as corners of the uterus.

A swingable mechanism 11-4 of the expanding end 11 configured to adjust the orientation of the working portion is arranged on the handle 52. Since the swingable mechanism 11-4 is arranged on the handle 52, the working direction of the working portion of the expanding end 11 can be adjusted very conveniently outside a body during the clinical use. Therefore, the operation is simpler.

The observation direction of the camera 22-1 of the lens module 22 and the orientation of the working portion of the expanding end 11 are both adjustable. During actual application, a clinician may adjust only the observation direction of the camera 22-1 or the orientation of the working portion of the expanding end 11 as needed, or may adjust both the observation direction of the camera 22-1 and the orientation of the working portion of the expanding end 11.

When the observation direction of the camera 22-1 and the orientation of the working portion of the expanding end 11 are both adjusted, the adjustment mechanism 22-11 and the swingable mechanism 11-4 may be combined into a single adjustment mechanism arranged on the handle 52.

The cavity expanding mechanism 1 further includes a sheath 12, the expanding mesh 11-1 is compressible into the sheath 12, and the expanding mesh 11-1 is completely or substantially restored to a shape before the compression after the restraint of the sheath 12 is removed. The sheath 12 includes an inner sheath 12-1 and an outer sheath 12-2, and the expanding mesh 11-1 is compressed into the outer sheath 12-2.

The expanding mesh 11-1 is compressible into the sheath 12, and the expanding mesh 11-1 is completely or substantially restored to a shape before the compression after the restraint of the sheath 12 is removed. During clinical use, the expanding mesh 11-1 may be folded into the sheath 12, and after entering the cavity, the sheath 12 is retracted to release the expanding mesh 11-1, so that the expanding mesh 11-1 is unfolded to expand the cavity in front of or around the camera 22-1, thereby expanding the field of view. In this way, during clinical use, examination and surgical sites and surrounding tissues can be observed more effectively, and the clinical observation effect is very desirable. A scraping wire 11-31 of the expanding mesh 11-1 may be made of materials of various shapes such as a wire, a thin tube, or a sheet. Moreover, the expanding mesh 11-1 may be configured as various geometric shapes such as an ellipsoid or a pear shape according to a shape of the cavity, so that the expanding mesh can adapt to the shape of the cavity more desirably, and is especially suitable for corners in the cavity that are difficult to remove.

The visualized medical system 100 with a cavity expanding device further includes a negative-pressure aspirating mechanism 4. The negative-pressure aspirating mechanism 4 can quickly aspirate tissue fluids such as watery blood generated during examination or surgery out of the body, so that clearer observation is realized during clinical use.

The negative-pressure aspirating mechanism 4 of the visualized medical system 100 with a cavity expanding device includes an aspirating inlet 41, an aspirating outlet 42, and an aspirating channel 43, the sheath 12 of the visualized medical system 100 with a cavity expanding device is a tube with two cavities including one cavity provided with the observation mechanism 2 and the other cavity constituting or provided with the aspirating channel 43, the aspirating inlet 41 is arranged at a front end of the sheath 12, and the aspirating outlet 42 of the negative-pressure aspirating mechanism 4 is arranged at a rear end of the sheath 12. The design of the double-cavity channel can isolate the circuit system 3 from liquids such as watery blood and tissue fluids that may be generated during surgery, so that safety hazards such as a short circuit and electric leakage can be avoided during use. In this way, the clinical use process is safer.

A negative-pressure control switch 44 configured to control a negative-pressure status is arranged on the negative-pressure aspirating mechanism 4. The negative-pressure control switch 44 can be used to turn on or off the negative-pressure aspirating mechanism 4, and can be used to adjust a negative-pressure aspirating force of the negative-pressure aspirating mechanism 4 according to an amount of stripped tissues, watery blood, and tissue fluids in the uterus during the surgery. During clinical use, a doctor can easily adjust the negative-pressure aspirating force as required, thereby avoiding a failure of aspirating the stripped tissues in time as a result of an excessively small negative-pressure aspirating force and accidental damage to tissues such as endometrium as a result of an excessively large negative-pressure aspirating force.

The negative-pressure control switch 44 is arranged on the handle 52 of the operating mechanism 5. Since the negative-pressure control switch 44 is arranged on the handle 52, medical staff can perform operations with one hand during clinical use. Therefore, the use process is more convenient.

The visualized medical system 100 with a cavity expanding device further includes a flushing mechanism 6, and at least one water outlet 61 of the flushing mechanism 6 is located at a front end of the camera 22-1 of the lens module 22. During examination and surgery, the flushing mechanism 6 can flush the camera 22-1, and can remove watery blood in front of the camera 22-1 in time to keep the field of view clean. In addition, the flushing mechanism 6 can flush inside of the cavity, especially the surgical sites in time, so that the surgical or examination sites can be observed in time, thereby ensuring safety of the surgical or examination process. In this way, a clinical surgery process is safer.

The sheath 12 of the visualized medical system 100 with a cavity expanding device is a tube with three cavities including a first cavity provided with the observation mechanism 2, a second cavity constituting or provided with the aspirating channel 43, and a third cavity constituting a water inlet pipe 63 of the flushing mechanism 6 or configured for entry and exit of a surgical instrument, and a water inlet 62 of the flushing mechanism 6 is arranged at a rear end of the sheath 12. The design of the three-cavity channel not only can isolate and protect the circuit system 3, but also can separate a flushing channel from the aspirating channel. In this way, it is ensured that no tissue or fluid that is aspirated out returns to the uterus during the flushing, and the use process is more efficient and safer.

A flushing switch 64 configured to control a flushing water flow is arranged on the flushing mechanism 6. The flushing switch 64 can be used to turn on and off the flushing mechanism 6, and can be used to control a water amount and a flushing pressure of the flushing water flow. During clinical use, a clinician may turn on or off the flushing mechanism 6 according to actual requirements during the surgery, and select a proper volume and a proper pressure of the flushing water flow as required. In this way, the clinical use process is more convenient.

The flushing switch 64 is arranged on the handle 52 of the operating mechanism 5. Since the flushing switch 64 is arranged on the handle 52, medical staff can perform operations with one hand during clinical use. Therefore, the use process is more convenient.

The expanding mesh 11-1 of the cavity expanding mechanism 1 is a woven mesh made of a wire. The design of the soft woven mesh causes less damage to the cavity.

The expanding mesh 11-1 has varying geometrical shapes. The expanding mesh 11-1 may be configured as varying geometric shapes such as a sphere, an ellipsoid, or a pear shape according to a shape of the cavity.

The expanding mesh 11-1 is made of a medical elastic material. An elastic effect of the medical elastic material can provide an effective expanding force for the expanding mesh 11-1. In particular, when the expanding mesh 11-1 is released from the sheath 12, the shape of the expanding mesh 11-1 made of the elastic medical material can be easily restored. Moreover, the elastic force of the medical elastic material is a moderate force, so that a very desirable buffering effect can be provided during the expansion of the expanding mesh 11-1, thereby preventing accidental injury to the surrounding tissues or organs during the expansion of the expanding mesh 11-1.

The expanding mesh 11-1 is made of medical elastic stainless steel or medical titanium-nickel shape memory alloy. The applicant enumerates only the above two medical elastic materials. During actual application, those skilled in the art may select a different medical elastic material for manufacturing as required. All of these do not depart from the protection scope of this application.

The expanding mesh 11-1 is made of a medical transparent elastic polymer material. Since the expanding mesh 11-1 may alternatively be made of the medical transparent elastic polymer material, not only elastic buffering is ensured, but also an observation path of the camera 22-1 is not blocked at all, thereby realizing a more desirable observation effect.

A coating is arranged on a protection cover 20 of the observation mechanism 2. The coating may be a hydrophobic coating, so that liquids such as watery blood can quickly condense into water droplets on the protection cover 20 and then slide off. Alternatively, the coating may be a hydrophilic coating, so that liquids such as watery blood can quickly form a transparent water film on a surface of the protection cover 20 and do not block the camera.

The observation mechanism 2 includes at least two cameras 22-1. Since the lens module 22 includes the at least two cameras 22-1, a limited field of view of an existing single lens can be effectively resolved, and the field of view during the surgery can be effectively expanded. In particular, when the cameras 22-1 are respectively arranged at the front end of and on a side surface of the operating mechanism 5, a three-dimensional observation space can be formed around the operating mechanism 5 by superimposing the fields of view. In this way, the clinical observation effect is very desirable.

At least one of the cameras 22-1 is arranged at the front end 5-1 of the operating mechanism 5. The camera 22-1 arranged in front of the operating mechanism 5 can effectively observe an area in front of the operating mechanism 5.

At least one of the cameras 22-1 is arranged on a side surface 5-5 of the operating mechanism 5. The camera 22-1 arranged on the side surface 5-5 of the operating mechanism 5 can observe an area on the side surface of the operating mechanism 5.

Observation areas of two or more of the cameras 22-1 are contiguous or partially overlapped. After the observation areas of the cameras 22-1 respectively arranged at the front end 5-1 and on the side surface 5-5 of the operating mechanism 5 are connected to each other, a spatial field of view can be formed, so that the clinical observation effect is more desirable.

The observation mechanism 2 further includes an instrument channel 25. The instrument channel 25 may be further arranged on the observation mechanism 2, and the instrument channel 25 may be used as the water inlet pipe 63 for flushing the cavity, and may be further used as a channel for entry and exit of the surgical instrument. Therefore, various surgical operations such as tissue flushing, biopsy, electrocution, coagulation, and ablation may be performed during the observation.

An electrical interface 31 connected to a host is arranged on the circuit system 3. The visualized medical system 100 with a cavity expanding device is connected to the host and a power supply by the electrical interface 31.

The visualized medical system 100 with a cavity expanding device includes an electronic protection mechanism 30, and the electronic protection mechanism 30 is arranged on the outside of the observation module 2 and/or the circuit system 3 and configured to perform waterproof, gasproof, and insulation protection on electronic components of the observation module 2 and/or the circuit system 3. Since the electronic protection mechanism 30 arranged on the outside of the observation module 2 and/or the circuit system 3 can perform waterproof, gasproof, and insulation protection on the electronic components of the observation module 2 and/or the circuit system 3, the watery blood or the tissue fluids generated during the surgery can be prevented from affecting normal operation of the electronic components, thereby ensuring normal operation of the surgery. In this way, the clinical surgery process is safer.

The electronic protection mechanism 30 is a protection mechanism 30-1 arranged around the lighting module 21 and the lens module 22, or may be an insulating protective layer 30-2 arranged on the outside of the circuit system 3, or may be an insulating glue layer 30-3 arranged in the handle 52 to protect an image processing system 103.

A protection cover 20 is arranged in front of the lighting module 21 and the lens module 22, and an isolation seat 25 is arranged behind a side of the lighting module 21 and the lens module 22. The protection cover 20, the isolation seat 25, and the housing 24 form a closed space by bonding or the like to form the protection mechanism 30-1, so as to completely isolate the lighting module 21 and the lens module 22 from the human tissues, thereby ensuring that the watery blood or the tissue fluids generated during the surgery do not affect normal operation of the lighting module 21 and the lens module 22.

Since the waterproof and insulative insulating protective layer 30-2 is arranged on the outside of the circuit system 3, accidents such as a short circuit that may be caused by the watery blood or the tissue fluids generated during the surgery can be effectively avoided.

When the signal processing module 23 is arranged in the handle 52, glue may be filled around the signal processing module 23 to form the insulating glue layer 30-3, so as to completely seal and isolate the signal processing module 23 and the surrounding circuit system 3, thereby realizing waterproof, gasproof, and insulation protection.

The visualized medical system 100 with a cavity expanding device further includes an identification system 8. The identification system 8 can prompt a depth by which the operating mechanism 5 enters the human body. The identification system 8 may be a scale 81 arranged on the outside of the operating mechanism 5, or may be other identification means. The applicant does not enumerate examples herein. All of these do not deviate from the protection scope of this application.

The visualized medical system 100 with a cavity expanding device further includes a flushing system 106, a water inlet 106-1 of the flushing system 106 is connected to an infusion bottle or bag 7 by a water pipe 106-4, and a water outlet 106-2 of the flushing system 106 is connected to a water inlet 62 of a flushing mechanism 6 of the visualized medical system 100 with a cavity expanding device by the water pipe 106-4. The flushing system 106 can flush the visualized medical system 100 with a cavity expanding device and surrounding tissues in time during the examination or surgery, so as to ensure that the field of view is kept clean and that the surgical sites can be flushed in time, thereby ensuring clear observation during the examination or surgery. In this way, the clinical surgery process is safer.

The flushing system 106 is driven by a peristaltic pump 106-3. The peristaltic pump 106-3 can control a flow and a speed of incoming water more precisely. Certainly, during actual application, those skilled in the art may use a different driving device to drive the flushing system 106. All of these do not depart from the protection scope of this application.

The visualized medical system 100 with a cavity expanding device further includes a negative-pressure aspirator 105, and an aspirating outlet 42 of a negative-pressure aspirating mechanism 4 of the visualized medical system 100 with a cavity expanding device is connected to the negative-pressure aspirator 105. Since the visualized medical system 100 with a cavity expanding device has the negative-pressure aspirator 105 and the flushing system 106, a negative-pressure aspirating function and a flushing function are integrated into one device, so that a whole surgical process of direct-view abortion can be completed by only one device without requiring an external negative-pressure source and an external flushing system. In this way, restrictions and impact of an external environment on the surgical process are significantly reduced, and the visualized medical system with a cavity expanding device can have a very wide application range.

During clinical application, the observation mechanism 2 is placed in the expanding mechanism 1, the expanding mesh 11-3 is received in the sheath 12, the visualized medical system 100 with a cavity expanding device is placed into the body, the sheath 12 is retracted, so that the expanding mesh 11-3 is expanded, the cavity in front of the observation mechanism 2 is expanded, and the camera 22-1 can observe the surrounding tissues and organs, and when necessary, the surgical instrument is inserted into the body through the instrument channel 25 or the sheath 12 to perform surgical operations such as biopsy sampling, electrocution, electrocoagulation, and ablation. The camera 22-1 of the observation mechanism 2 and the cavity can be flushed in time by the flushing mechanism 6, and the liquids such as the tissue fluids, the water blood, and flushing fluids can be aspirated out of the body in time by the negative-pressure aspirating mechanism 4.

The visualized medical system with a cavity expanding device of the present invention includes the cavity expanding mechanism 1, the observation mechanism 2, the circuit system 3, the power supply system 104, the operating mechanism 5, and the display system 102. The cavity expanding mechanism 1 includes the expanding end 11. The expanding end 11 is arranged at the front end 5-1 of the operating mechanism 5, and is configured to expand the cavity in front of the observation mechanism 2 to expand the field of view of the observation mechanism 2. The observation mechanism 2 includes the lighting module 21, the lens module 22, the signal processing module 23, and the housing 24. The image and the video observed in the lens module 22 in the light field of the lighting module 21 are processed by the signal processing module 23, and then outputted to the display system 102 by the circuit system 3 for displaying. The lighting module 21, the lens module 22, and the circuit system 3 are connected and then mounted in the housing 24 in a sealed manner. Since the cavity in front of the observation mechanism 2 is expanded, a field of view of the lens module 22 is larger. Therefore, a clinical observation effect is more desirable. The visualized medical system with a cavity expanding device of the present invention can introduce a surgical instrument into a body from the sheath 12, and perform various surgical operations such as biopsy sampling, electrocution, electrocoagulation, and ablation during observation, which is very convenient for clinical use.

### BRIEF DESCRIPTION OF THE DRAWINGS

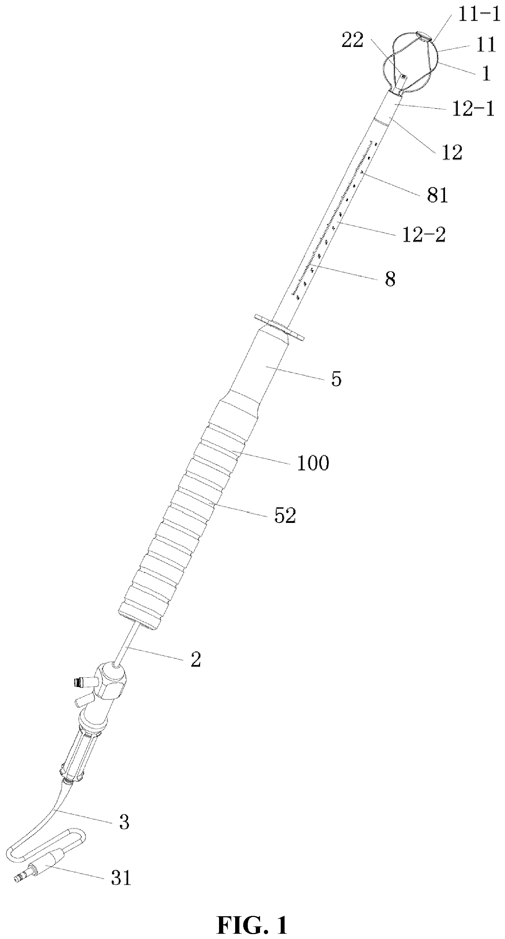

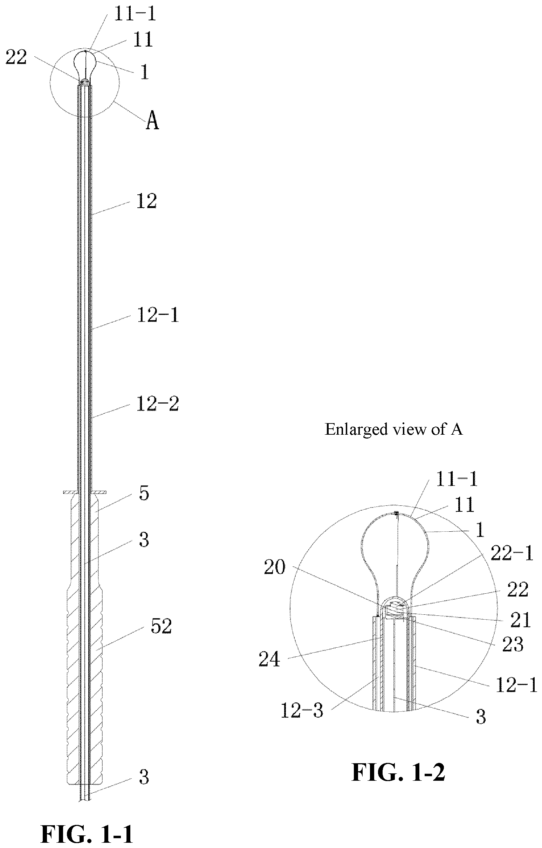

FIG. 1 is a schematic three-dimensional structural diagram of a visualized medical system with a cavity expanding device according to the present invention.
FIG. 1-1 is a cross-sectional view of FIG. 1.
FIG. 1-2 is an enlarged view of A in FIG. 1-1.
FIG. 2 is a schematic three-dimensional structural diagram of a visualized medical system with a cavity expanding device according to the present invention having a negative-pressure aspirating mechanism and a flushing mechanism.
FIG. 2-1 is a cross-sectional view of FIG. 2.
FIG. 2-2 is an enlarged view of B in FIG. 2-1.

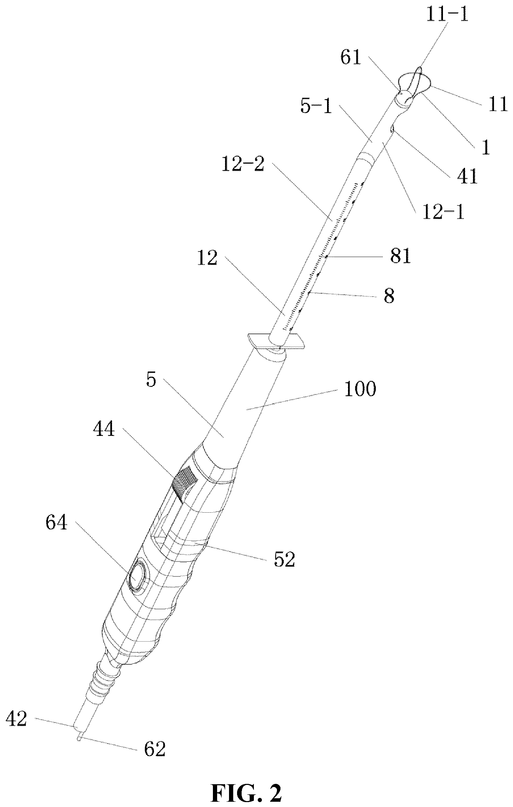

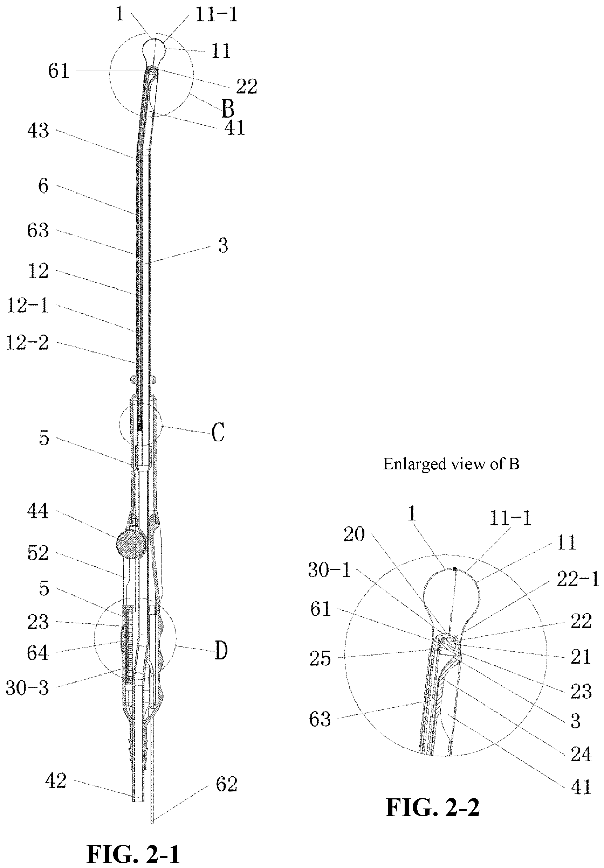

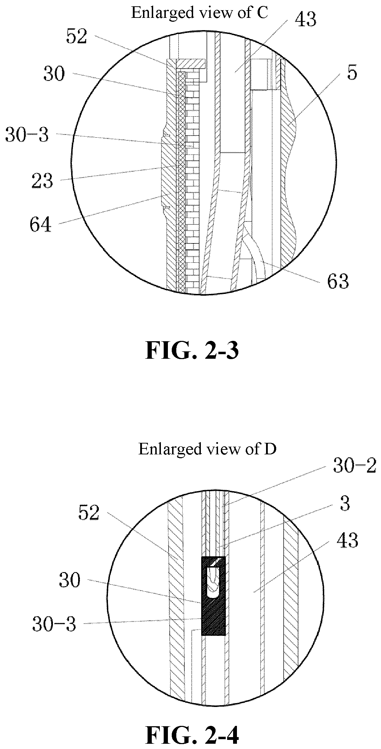

FIG. 2-3 is an enlarged view of C in FIG. 2-1.
FIG. 2-4 is an enlarged view of D in FIG. 2-1.

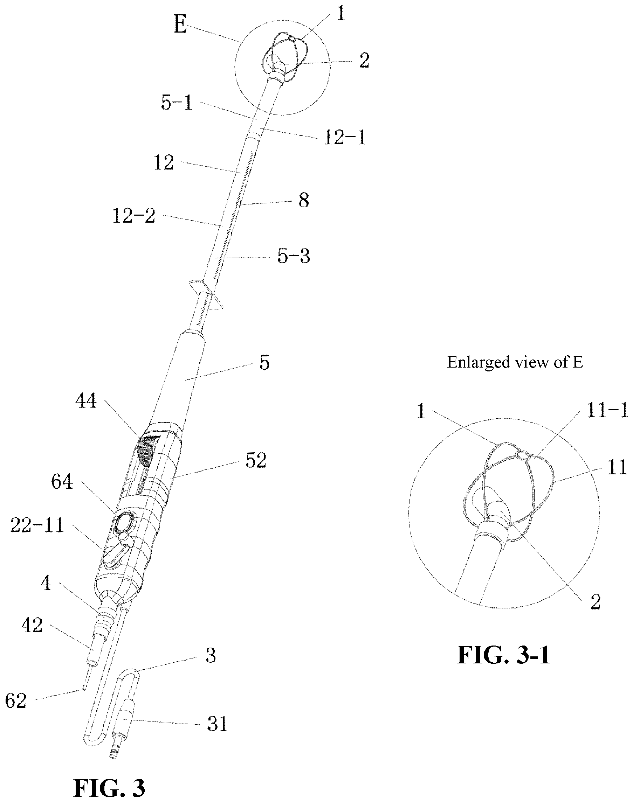

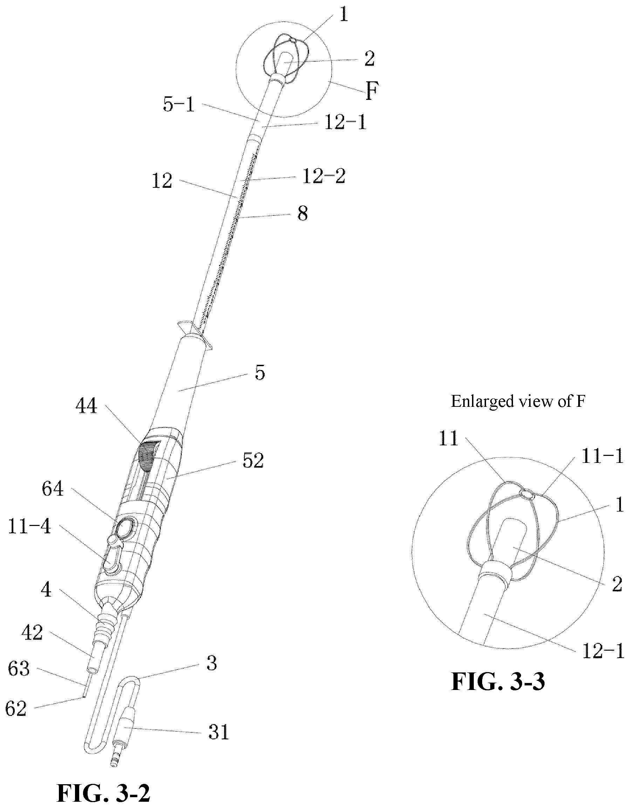

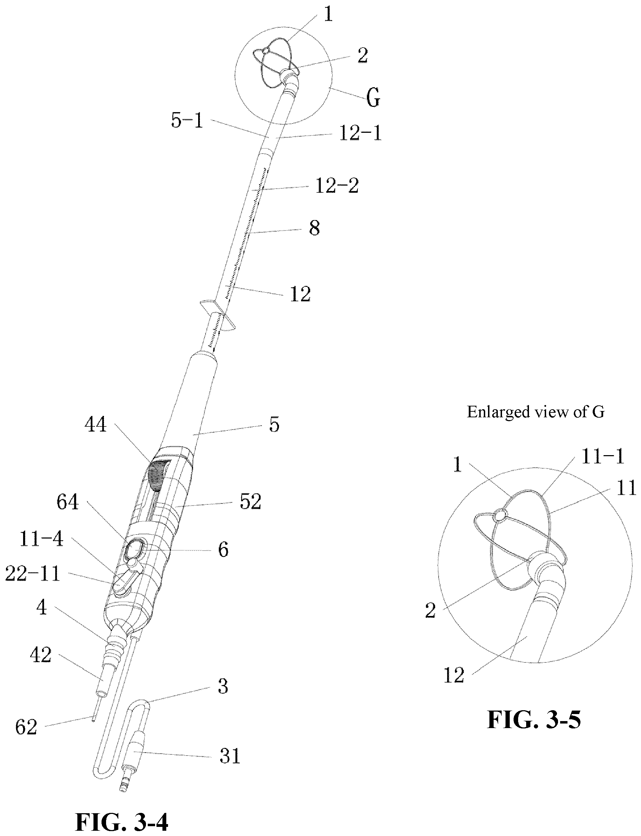

FIG. 3 is a schematic three-dimensional structural diagram of a visualized medical system with a cavity expanding device according to the present invention having an adjustable observation direction.
FIG. 3-1 is an enlarged view of E in FIG. 3.
FIG. 3-2 is a schematic three-dimensional structural diagram of the visualized medical system with a cavity expanding device according to the present invention having an adjustable expanding mesh orientation.
FIG. 3-3 is an enlarged view of F in FIG. 3-2.
FIG. 3-4 is a schematic three-dimensional structural diagram of the visualized medical system with a cavity expanding device according to the present invention having an observation direction and an expanding mesh orientation that are both adjustable.
FIG. 3-5 is an enlarged view of G in FIG. 3-4.

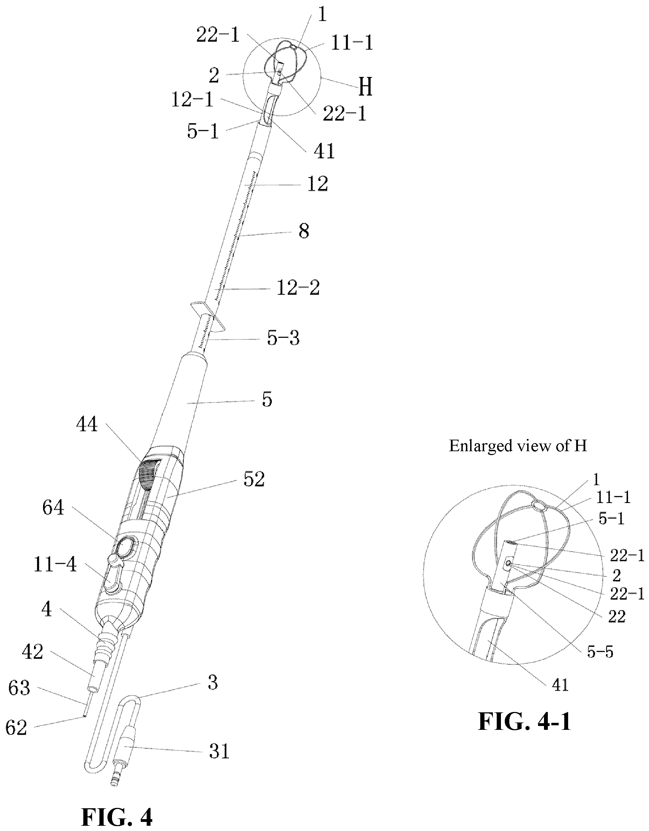

FIG. 4 is a schematic three-dimensional structural diagram of a visualized medical system with a cavity expanding device according to the present invention including two cameras.
FIG. 4-1 is an enlarged view of H in FIG. 4.

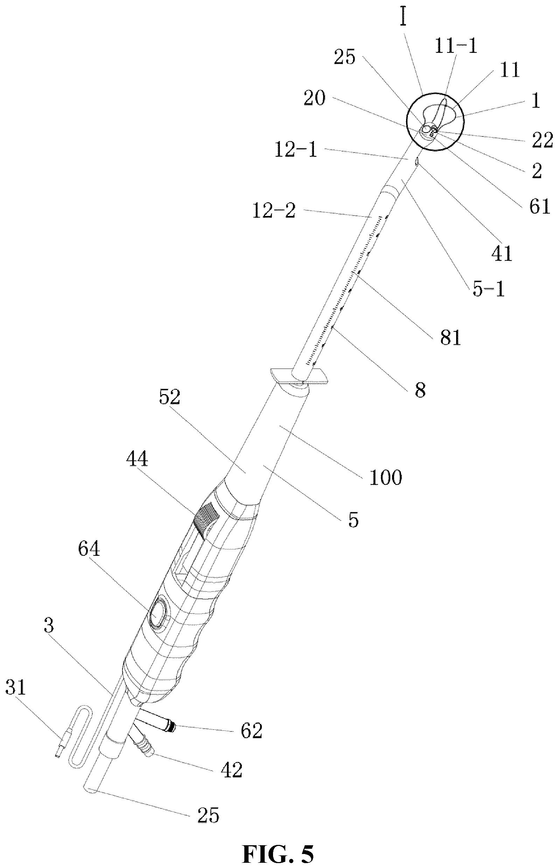

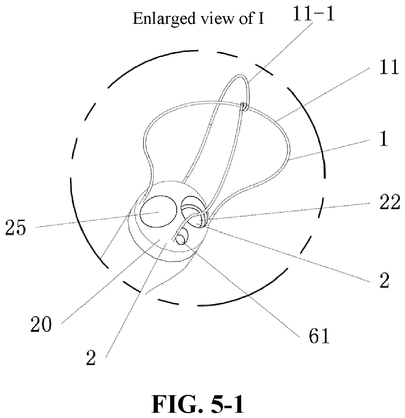

FIG. 5 is a schematic three-dimensional structural diagram of a visualized medical system with a cavity expanding device according to the present invention including an observation mechanism including an instrument channel.
FIG. 5-1 is an enlarged view of I in FIG. 5.

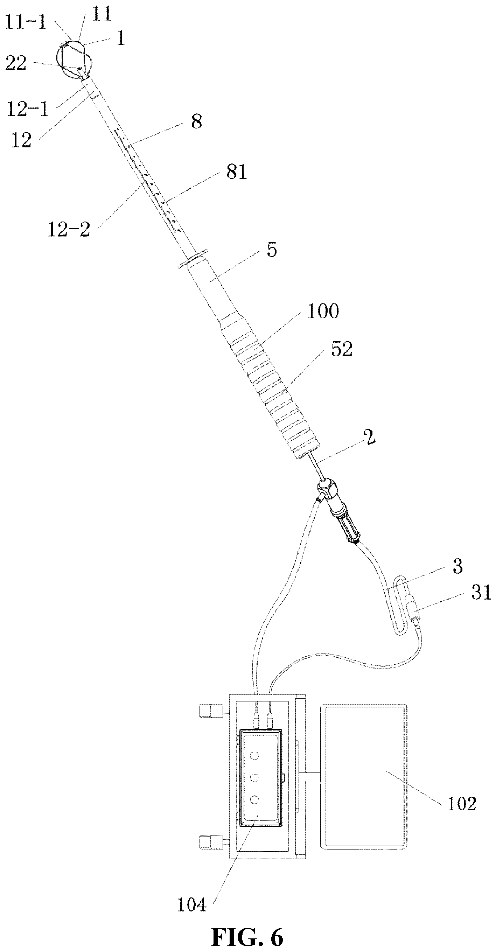

FIG. 6 is a working principle diagram of the visualized medical system with a cavity expanding device according to the present invention.

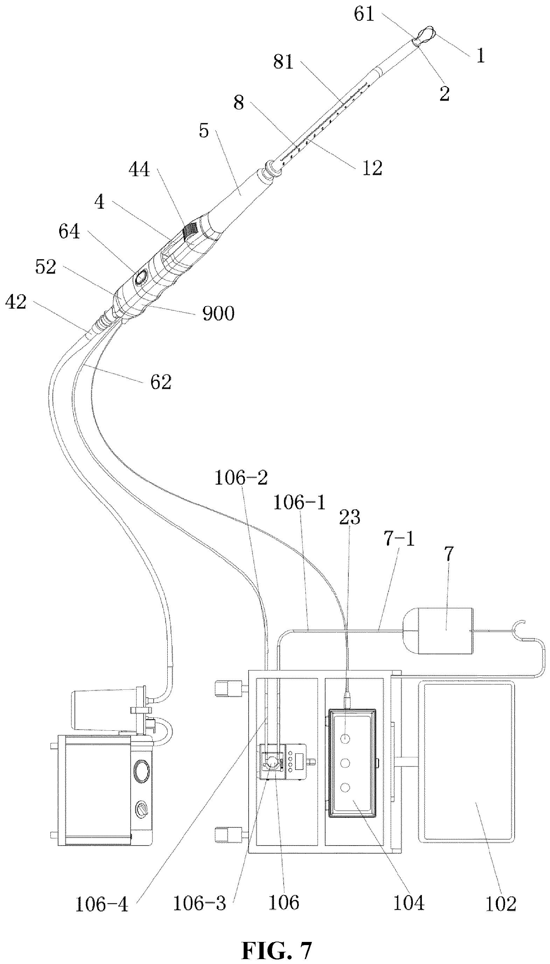

FIG. 7 is a working principle diagram of a visualized medical system with a cavity expanding device according to the present invention having a flushing system.

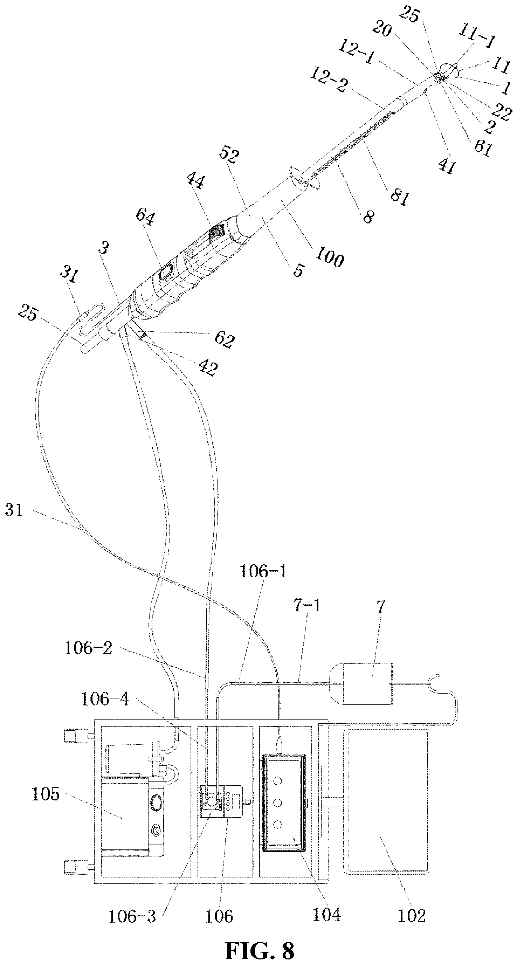

FIG. 8 is a working principle diagram of a visualized medical system with a cavity expanding device according to the present invention having a negative-pressure aspirator.

In the foregoing figures:
100: Visualized medical system with a cavity expanding device according to the present invention;
102: Display system, 104: Power supply system, 105: negative-pressure aspirator, 106: Flushing system;
1: Cavity expanding mechanism, 2: Observation mechanism, 3: Circuit system, 4: Negative-pressure aspirating mechanism, 5: Operating mechanism, 6: Flushing mechanism, 7: Infusion bottle or bag, 8: Identification system;
11: Expanding end, 11-1: Expanding mesh, 11-4: Swingable mechanism, 12: Sheath, 12-1: Inner sheath, 12-2: Outer sheath, 12-3: Channel;
20: Protection cover, 21: Lighting module, 22: Lens module, 23: Signal processing module, 24: Housing, 25: Isolation seat, 22-1: Camera, 22-11: Adjustment mechanism.
30-1: Protection mechanism, 30-2: Insulating protective layer, 30-3: Insulating glue layer;
31: Electric interface;
41: Aspirating inlet, 42: Aspirating outlet, 43: Aspirating channel, 44: Negative-pressure control switch;
5-1: Front end, 5-5: Side surface, 52: Handle;
61: Water outlet, 62: Water inlet, 63: Water inlet pipe, 64 Flushing switch;
7-1: Pipe, 81: Scale;
106-1: Water inlet, 106-2: Water outlet, 106-3: Peristaltic pump, 106-4; Water pipe.

### DESCRIPTION OF EMBODIMENTS

### Embodiment 1: visualized medical system with a cavity expanding device according to the present invention

Referring to FIG 1 to FIG. 1-2, the visualized medical system with a cavity expanding device in this embodiment includes a cavity expanding mechanism 1, an observation mechanism 2, a circuit system 3, a power supply system 104, an operating mechanism 5, and a display system 102.

Referring to FIG 1, the cavity expanding mechanism 1 includes an expanding end 11 and a sheath 12, and the sheath 12 is arranged at a rear end of the expanding end 11. The sheath 12 includes an inner sheath 12-1 and an outer sheath 12-2, and the expanding mesh 11-1 may be compressed into the outer sheath 12-2.

The expanding end 11 is arranged at a front end 5-1 of the operating mechanism 5, and is configured to expand a cavity in front of the observation mechanism 2 to expand a field of view of the observation mechanism 2.

In this embodiment, the expanding end 11 is an expanding mesh 11-1. A camera 22-1 may directly observe a situation behind the expanding mesh 11-1 through mesh gaps. Therefore, even if the expanding mesh 11-1 is not made of a transparent material, full observation of a surgical process and panoramic observation of a surgical area can be realized.

The observation mechanism 2 includes a lighting module 21, a lens module 22, a signal processing module 23, and a housing 24. An image and a video observed in the lens module 22 in a light field of the lighting module 21 are processed by the signal processing module 23, and then outputted to the display system 102 by the circuit system 3 for displaying. The lighting module 21, the lens module 22, and the circuit system 3 are connected and then mounted in the housing 24 in a sealed manner.

Referring to FIG. 6, the observation mechanism 2 is connected to the display system 102 by the circuit system 3 and the power supply system 104.

Referring to FIG. 1-1 and FIG. 1-2, in this embodiment, the observation mechanism 2 and the cavity expanding mechanism 1 are arranged separately.

The observation mechanism 2 is an independent sealing device that can move back and forth in a channel 12-3 of the sheath 12 of the cavity expanding mechanism 1. With the solution of independent arrangement. the observation mechanism 2 can be fixed at the rear end of or inside the expanding mesh 11-1 of the cavity expanding mechanism 1 as required, which is very flexible for clinical use. During clinical use, after observation is completed, the observation mechanism 2 is removed. The observation mechanism 2 can be reused after disinfection and sterilization, and only the cavity expanding mechanism 1 is used once. In this way, the clinical costs can be significantly reduced.

The observation mechanism 2 and the cavity expanding mechanism 1 may alternatively be arranged as a whole. In this case, the sheath 12 of the cavity expanding mechanism 1 constitutes the housing 24. The lighting module 21 and the lens module 22 of the observation mechanism 2 and the circuit system 3 are sealed in the sheath 12. During clinical use, the visualized medical system 100 with a cavity expanding device may be directly placed into a body for observation, so that a clinical use process is simple and convenient.

In this embodiment, the camera 22-1 of the lens module 22 is arranged at a rear end of the expanding mesh 11-1, and the expanding mesh 11-1 is located within a field of view of the camera 22-1.

The technical solution of arranging the camera 22-1 at the rear end can ensure that the expanding mesh 11-1 is completely within the field of view of the camera 22-1. In addition, since an observation angle of the camera 22-1 is oriented toward a front of a surgical or examination area, there is no observation blind in the above process. Therefore, the technical solution of arranging the camera at the rear end causes processes of clinical examination and surgery to be safer and more effective.

Referring to FIG. 1, in this embodiment, the expanding mesh 11-1 is a mesh-like structure formed by winding two wires. During actual application, the expanding mesh 11-1 of the cavity expanding mechanism 1 is a woven mesh made of a wire. The design of the soft woven mesh causes less damage to the cavity.

The expanding mesh 11-1 has varying geometrical shapes. The expanding mesh 11-1 may be configured as varying geometric shapes such as a sphere, an ellipsoid, or a pear shape according to a shape of the cavity.

The expanding mesh 11-1 is made of a medical elastic material. An elastic effect of the medical elastic material can provide an effective expanding force for the expanding mesh 11-1. In particular, when the expanding mesh 11-1 is released from the sheath 12, the shape of the expanding mesh 11-1 made of the elastic medical material can be easily restored. Moreover, the elastic force of the medical elastic material is a moderate force, so that a very desirable buffering effect can be provided during the expansion of the expanding mesh 11-1, thereby preventing accidental injury to the surrounding tissues or organs during the expansion of the expanding mesh 11-1.

The expanding mesh 11-1 is made of medical elastic stainless steel or medical titanium-nickel shape memory alloy. The applicant enumerates only the above two medical elastic materials. During actual application, those skilled in the art may select a different medical elastic material for manufacturing as required. All of these do not depart from the protection scope of this application.

The expanding mesh 11-1 is made of a medical transparent elastic polymer material. Since the expanding mesh 11-1 may alternatively be made of the medical transparent elastic polymer material, not only elastic buffering is ensured, but also an observation path of the camera 22-1 is not blocked at all, thereby realizing a more desirable observation effect.

An electrical interface 31 connected to a host is arranged on the circuit system 3. The visualized medical system 100 with a cavity expanding device is connected to the host and a power supply by the electrical interface 31.

The visualized medical system 100 with a cavity expanding device further includes an identification system 8. The identification system 8 can prompt a depth by which the operating mechanism 5 enters the human body. The identification system 8 may be a scale 81 arranged on the outside of the operating mechanism 5, or may be other identification means. The applicant does not enumerate examples herein. All of these do not deviate from the protection scope of this application.

According to the visualized medical system with a cavity expanding device in this embodiment, the expanding end 11 of the cavity expanding mechanism 1 is arranged at a front end of the camera 22-1 of the observation mechanism 2, and the expanding end 11 can expand the cavity in front of the observation mechanism 2 to expand the field of view of the observation mechanism 2. This is especially suitable for examination and surgery processes such as hysteroscopy or colonoscopy. During use, the visualized medical system with a cavity expanding device of the present invention is inserted into the cavity, the expanding end 11 expands the cavity, the observation mechanism 2 observes the expanded area, and image and video signals observed in the lens module 22 are outputted to the display system 102 by the circuit system 3 for displaying. Since the cavity in front of the observation mechanism 2 is expanded, a field of view of the lens module 22 is larger. Therefore, a clinical observation effect is more desirable.

### Embodiment 2: visualized medical system with a cavity expanding device according to the present invention having a negative-pressure aspirating mechanism and a flushing mechanism

Referring to FIG. 2 to FIG. 2-4, a difference between this embodiment and Embodiment 1 is that, the visualized medical system 100 with a cavity expanding device in this embodiment further includes a negative-pressure aspirating mechanism 4 and a flushing mechanism 6.

Referring to FIG. 2-1, the sheath 12 includes an inner sheath 12-1 and an outer sheath 12-2, and the expanding mesh 11-1 may be compressed into the outer sheath 12-2. The inner sheath 12-1 is a tube with three cavities including a first cavity provided with the observation mechanism 2, a second cavity constituting or provided with an aspirating channel 43, and a third cavity constituting a water inlet pipe 63 of the flushing mechanism 6 or configured for entry and exit of a surgical instrument.

The negative-pressure aspirating mechanism 4 includes an aspirating inlet 41, an aspirating outlet 42, and the aspirating channel 43. The aspirating inlet 41 is arranged at a front end of the inner sheath 12-1, and the aspirating outlet 42 of the negative-pressure aspirating mechanism 4 is arranged at a rear end of the inner sheath 12-1. The design of the double-cavity channel can isolate the circuit system 3 from liquids such as watery blood and tissue fluids that may be generated during surgery, so that safety hazards such as a short circuit and electric leakage can be avoided during use. In this way, the clinical use process is safer.

A negative-pressure control switch 44 configured to control a negative-pressure status is arranged on the negative-pressure aspirating mechanism 4. The negative-pressure control switch 44 can be used to turn on or off the negative-pressure aspirating mechanism 4, and can be used to adjust a negative-pressure aspirating force of the negative-pressure aspirating mechanism 4 according to an amount of stripped tissues, watery blood, and tissue fluids in the uterus during the surgery. During clinical use, a doctor can easily adjust the negative-pressure aspirating force as required, thereby avoiding a failure of aspirating the stripped tissues in time as a result of an excessively small negative-pressure aspirating force and accidental damage to tissues such as endometrium as a result of an excessively large negative-pressure aspirating force.

The negative-pressure control switch 44 is arranged on the handle 52 of the operating mechanism 5. Since the negative-pressure control switch 44 is arranged on the handle 52, medical staff can perform operations with one hand during clinical use. Therefore, the use process is more convenient.

The flushing mechanism 6 includes a water outlet 61, a water inlet 62, and a water inlet pipe 63.

Referring to FIG. 2-1 and FIG. 2-2, at least one water outlet 61 of the flushing mechanism 6 is located at a front end of the camera 22-1 of the lens module 22. During examination and surgery, the flushing mechanism 6 can flush the camera 22-1, and can remove watery blood in front of the camera 22-1 in time to keep the field of view clean. In addition, the flushing mechanism 6 can flush inside of the cavity, especially the surgical sites in time, so that the surgical or examination sites can be observed in time, thereby ensuring safety of the surgical or examination process. In this way, a clinical surgery process is safer.

The water inlet 62 of the flushing mechanism 6 is arranged at a rear end of the inner sheath 12-1. The design of the three-cavity channel not only can isolate and protect the circuit system 3, but also can separate a flushing channel from the aspirating channel. In this way, it is ensured that no tissue or fluid that is aspirated out returns to the uterus during the flushing, and the use process is more efficient and safer.

A flushing switch 64 configured to control a flushing water flow is arranged on the flushing mechanism 6. The flushing switch 64 can be used to turn on and off the flushing mechanism 6, and can be used to control a water amount and a flushing pressure of the flushing water flow. During clinical use, a clinician may turn on or off the flushing mechanism 6 according to actual requirements during the surgery, and select a proper volume and a proper pressure of the flushing water flow as required. In this way, the clinical use process is more convenient.

The flushing switch 64 is arranged on the handle 52 of the operating mechanism 5. Since the flushing switch 64 is arranged on the handle 52, medical staff can perform operations with one hand during clinical use. Therefore, the use process is more convenient.

Referring to FIG. 2-1, FIG. 2-3, and FIG. 2-4, the visualized medical system 100 with a cavity expanding device includes an electronic protection mechanism 30, and the electronic protection mechanism 30 is arranged on the outside of the observation module 2 and/or the circuit system 3 and configured to perform waterproof, gasproof, and insulation protection on electronic components of the observation module 2 and/or the circuit system 3. Since the electronic protection mechanism 30 arranged on the outside of the observation module 2 and/or the circuit system 3 can perform waterproof, gasproof, and insulation protection on the electronic components of the observation module 2 and/or the circuit system 3, the watery blood or the tissue fluids generated during the surgery can be prevented from affecting normal operation of the electronic components, thereby ensuring normal operation of the surgery. In this way, the clinical surgery process is safer.

The electronic protection mechanism 30 is a protection mechanism 30-1 arranged around the lighting module 21 and the lens module 22, or may be an insulating protective layer 30-2 arranged on the outside of the circuit system 3, or may be an insulating glue layer 30-3 arranged in the handle 52 to protect an image processing system 103.

A protection cover 20 is arranged in front of the lighting module 21 and the lens module 22, and an isolation seat 25 is arranged behind a side of the lighting module 21 and the lens module 22. The protection cover 20, the isolation seat 25, and the housing 24 form a closed space by bonding or the like to form the protection mechanism 30-1, so as to completely isolate the lighting module 21 and the lens module 22 from the human tissues, thereby ensuring that the watery blood or the tissue fluids generated during the surgery do not affect normal operation of the lighting module 21 and the lens module 22.

Since the waterproof and insulative insulating protective layer 30-2 is arranged on the outside of the circuit system 3, accidents such as a short circuit that may be caused by the watery blood or the tissue fluids generated during the surgery can be effectively avoided.

When the signal processing module 23 is arranged in the handle 52, glue may be filled around the signal processing module 23 to form the insulating glue layer 30-3, so as to completely seal and isolate the signal processing module 23 and the surrounding circuit system 3, thereby realizing waterproof, gasproof, and insulation protection

In this embodiment, flushing mechanism 6 can flush the camera 22-1 and the cavity in time, and the liquids such as the tissue fluids, the water blood, and flushing fluids generated during the examination or surgery can be aspirated out of the body in time by the negative-pressure aspirating mechanism 4. In this way, the observation is more clear and safer during the clinical use.

### Embodiment 3: visualized medical system with a cavity expanding device according to the present invention having an adjustable observation direction.

Referring to FIG. 3 to FIG. 3-5, a difference between this embodiment and Embodiment 1 is that, the camera 22-1 of the lens module 22 in this embodiment is arranged inside the scraper 11.

In this embodiment, the camera 22-1 of the lens module 22 is arranged inside the scraper 11.

Referring to FIG. 3 and FIG. 3-1, an observation direction of the camera 22-1 of the lens module 22 is adjustable. By adjusting the observation direction of the camera 22-1, the surrounding tissues can be observed in all directions. In this way, the clinical observation effect is more comprehensive.

The adjustment mechanism 22-11 of the camera 22-1 configured to adjust the observation direction is arranged on the handle 52 of the operating mechanism 5. Since the adjustment mechanism 22-11 is arranged on the handle 52, the observation direction of the camera 22-1 can be adjusted very conveniently outside a body during the clinical use. Therefore, the operation is simpler.

Referring to FIG. 3-2 and FIG. 3-3, an orientation of a working portion of the expanding end 11 is adjustable. The orientation of the working portion of the expanding end 11 may be adjusted as required, so that the expanding end can adapt to a structure and a shape of a surrounding cavity more effectively, and a more desirable clinical use effect can be achieved, especially for areas that are difficult to remove or observe such as corners of the uterus.

A swingable mechanism 11-4 of the expanding end 11 configured to adjust the orientation of the working portion is arranged on the handle 52. Since the swingable mechanism 11-4 is arranged on the handle 52, the working direction of the working portion of the scraper 11 can be adjusted very conveniently outside a body during the clinical use. Therefore, the operation is simpler.

Referring to FIG. 3-4 and FIG. 3-5, the observation direction of the camera 22-1 of the lens module 22 and the orientation of the working portion of the expanding end 11 are both adjustable. During actual application, a clinician may adjust only the observation direction of the camera 22-1 or the orientation of the working portion of the expanding end 11 as needed, or may adjust both the observation direction of the camera 22-1 and the orientation of the working portion of the expanding end 11.

When the observation direction of the camera 22-1 and the orientation of the working portion of the expanding end 11 are both adjusted, the adjustment mechanism 22-11 and the swingable mechanism 11-4 may be combined into a single adjustment mechanism arranged on the handle 52.

In this embodiment, since the observation direction of the camera 22-1 and the orientation of the working portion of the expanding mesh 11 are adjustable, the clinical use effect is more desirable for corners that are difficult to remove and observe during the clinical use.

### Embodiment 4: visualized medical system with a cavity expanding device according to the present invention including a plurality of cameras

Referring to FIG. 4 and FIG. 4-1, a difference between this embodiment and Embodiment 1 is that, the lens module 22 in this embodiment includes at least two cameras 22-1.

Since the lens module 22 includes the at least two cameras 22-1, a limited field of view of an existing single lens can be effectively resolved, and the field of view during the surgery can be effectively expanded. In particular, when the cameras 22-1 are respectively arranged at the front end of and on a side surface of the operating mechanism 5, a three-dimensional observation space can be formed around the operating mechanism 5 by superimposing the fields of view. In this way, the clinical observation effect is very desirable.

At least one of the cameras 22-1 is arranged at the front end 5-1 of the operating mechanism 5. The camera 22-1 arranged in front of the operating mechanism 5 can effectively observe an area in front of the operating mechanism 5.

At least one of the cameras 22-1 is arranged on a side surface 5-5 of the operating mechanism 5. The camera 22-1 arranged on the side surface 5-5 of the operating mechanism 5 can observe an area on the side surface of the operating mechanism 5.

Observation areas of two or more of the cameras 22-1 are contiguous or partially overlapped. After the observation areas of the cameras 22-1 respectively arranged at the front end 5-1 and on the side surface 5-5 of the operating mechanism 5 are connected to each other, a spatial field of view can be formed, so that the clinical observation effect is more desirable.

In this embodiment, since the lens module 22 includes the at least two cameras 22-1, a limited field of view of an existing single lens can be effectively resolved, and the field of view during the surgery can be effectively expanded. In particular, when the cameras 22-1 are respectively arranged at the front end of and on the side surface of the operating mechanism 5, a three-dimensional observation space can be formed around the operating mechanism 5 by superimposing the fields of view. In this way, the clinical observation effect is very desirable.

### Embodiment 5: visualized medical system with a cavity expanding device according to the present invention including an instrument channel

Referring to FIG. 5 and FIG. 5-1, a difference between this embodiment and Embodiment 1 is that, the observation mechanism 2 in this embodiment includes an instrument channel 25.

Referring to FIG. 5 and FIG. 5-1, the instrument channel 25 and the water inlet pipe 63 are arranged on the observation mechanism 2. The surgical instrument can enter the body through the instrument channel 25, and perform various surgical operations such as biopsy sampling, electrocution, electrocoagulation, and laser ablation as required. The water outlet 61 of the water inlet pipe 63 is arranged near the camera 22-1, so that the camera 22-1 and the surrounding cavity tissues can be flushed in time. The liquids such as the tissue fluids, the water blood, and the flushing fluids generated during the flushing and the surgery can be aspirated out of the body in time by the negative-pressure aspirating mechanism 4.

The visualized medical system with a cavity expanding device in this embodiment can perform various surgical operations as required while performing effective observation, and can have a wider clinical application range.

### Embodiment 6: visualized medical system with a cavity expanding device according to the present invention including a flushing system.

Referring to FIG. 7, a difference between the visualized medical system with a cavity expanding device in this embodiment and that in Embodiment 1 is that, the visualized medical system with a cavity expanding device in this embodiment further includes a flushing system 106.

A water inlet 106-1 of the flushing system 106 is connected to an infusion bottle or bag 7 by a water pipe 106-4, and a water outlet 106-2 of the flushing system 106 is connected to a water inlet 62 of a flushing mechanism 6 of the visualized medical system with a cavity expanding device by the water pipe 106-4. The flushing system 106 can flush the visualized medical system with a cavity expanding device having the electronic protection mechanism and surrounding tissues in time during the surgery, so as to ensure that the field of view is kept clean and that the surgical sites can be flushed in time. In this way, it can be determined by observation in time whether embryonic tissues have been completely removed from an implantation site, and complete removal of the embryonic tissues during the abortion is ensured, thereby effectively avoiding incomplete abortion. Therefore, the clinical surgery process is safer.

Referring to FIG. 10, the flushing system 106 is driven by a peristaltic pump 106-3. The peristaltic pump 106-3 can control a flow and a speed of incoming water more precisely. Certainly, during actual application, those skilled in the art may use a different driving device to drive the flushing system 106. All of these do not depart from the protection scope of this application.

In this embodiment, the flushing system 106 can flush the visualized medical system with a cavity expanding device and surrounding tissues in time during the surgery, so as to ensure that the field of view is kept clean and that the surgical sites can be flushed in time. In this way, the clinical use is safer.

### Embodiment 7: visualized medical system with a cavity expanding device according to the present invention including a negative-pressure aspirator

Referring to FIG. 8, a difference between the visualized medical system with a cavity expanding device in this embodiment and that in Embodiment 6 is that, the visualized medical system with a cavity expanding device in this embodiment further includes a negative-pressure aspirator 105.

In this embodiment, an aspirating outlet 42 of the negative-pressure aspirating mechanism 4 of the visualized medical system with a cavity expanding device is connected to the negative-pressure aspirator 105. A water inlet 106-1 of the flushing system 106 is connected to an infusion bottle or bag 7 by a water pipe 7-1, and a water outlet 106-2 of the flushing system 106 is connected to a water inlet 62 of a flushing mechanism 6 by the water pipe 7-1.

During clinical use, the aspirating outlet 42 of the negative-pressure aspirating mechanism 4 is connected to the negative-pressure aspirator 105. During the surgery, the negative-pressure aspirator 105 can continuously aspirate the tissues stripped and the watery blood generated during the surgery out of the body.

Since the visualized medical system with a cavity expanding device in this embodiment has the negative-pressure aspirator 105 and the flushing system 106, a negative-pressure aspirating function and a flushing function are integrated into one device, so that a whole surgical process of direct-view abortion can be completed by only one device without requiring an external negative-pressure source and an external flushing system. In this way, restrictions and impact of an external environment on the surgical process are significantly reduced, and the visualized medical system with a cavity expanding device can have a very wide application range.

It should be noted that the structure disclosed and described in this specification may be replaced with another structure with the same effect. In addition, the embodiments described in the present invention are not the only structure of implementing the present invention. Although exemplary embodiments of the present invention have been introduced and described in this specification, it should be understood by a person skilled in the art that the embodiments are merely described by way of example, and a person skilled in the art may make various changes, improvements, and replacements without departing from the present invention. Therefore, the protection scope of the present invention should be defined in accordance with the spirit and scope of the claims appended to the present invention.

## Claims

1. A visualized medical system with a cavity expanding device, wherein the visualized medical system (100) with a cavity expanding device comprises a cavity expanding mechanism (1), an observation mechanism (2), a circuit system (3), a power supply system (104), an operating mechanism (5), and a display system (102), wherein
A. the cavity expanding mechanism (1) comprises an expanding end (11), and the expanding end (11) is arranged at a front end (5-1) of the operating mechanism (5), and is configured to expand a cavity in front of the observation mechanism (2) to expand a field of view of the observation mechanism (2);
B. the observation mechanism (2) comprises a lighting module (21), a lens module (22), a signal processing module (23), and a housing (24), wherein an image and a video observed in the lens module (22) in a light field of the lighting module (21) are processed by the signal processing module (23), and then outputted to the display system (102) by the circuit system (3) for displaying, and the lighting module (21), the lens module (22), and the circuit system (3) are connected and then mounted in the housing (24) in a sealed manner; and
C. the observation mechanism (2) is connected to the display system (102) by the circuit system (3) and the power supply system (104).

2. The visualized medical system with a cavity expanding device according to claim 1, wherein the expanding end (11) is an expanding mesh (11-1).

3. The visualized medical system with a cavity expanding device according to claim 1, wherein a camera (22-1) of the lens module (22) is arranged at a rear end of the expanding mesh (11-1), and the expanding mesh (11-1) is located within a field of view of the camera (22-1).

4. The visualized medical system with a cavity expanding device according to claim 1, wherein a camera (22-1) of the lens module (22) is arranged inside the expanding mesh (11-1).

5. The visualized medical system with a cavity expanding device according to claim 1, wherein an observation direction of a camera (22-1) of the lens module (22) is adjustable.

6. The visualized medical system with a cavity expanding device according to claim 5, wherein an adjustment mechanism (22-11) of the camera (22-1) configured to adjust the observation direction is arranged on a handle (52) of the operating mechanism (5).

7. The visualized medical system with a cavity expanding device according to claim 1, wherein an orientation of a working portion of the expanding end (11) is adjustable.

8. The visualized medical system with a cavity expanding device according to claim 7, wherein a swingable mechanism (11-4) of the expanding end (11) configured to adjust the orientation of the working portion is arranged on the handle (52).

9. The visualized medical system with a cavity expanding device according to claim 1, wherein the observation direction of the camera (22-1) of the lens module (22) and the orientation of the working portion of the expanding end (11) are both adjustable.

10. The visualized medical system with a cavity expanding device according to claim 1, wherein the cavity expanding mechanism (1) further comprises a sheath (12), the expanding mesh (11-1) is compressible into the sheath (12), and the expanding mesh (11) is completely or substantially restored to a shape before the compression after the restraint of the sheath (12) is removed.

11. The visualized medical system with a cavity expanding device according to claim 1, wherein the visualized medical system (100) with a cavity expanding device further comprises a negative-pressure aspirating mechanism (4).

12. The visualized medical system with a cavity expanding device according to claim 11, wherein the negative-pressure aspirating mechanism (4) of the visualized medical system (100) with a cavity expanding device comprises an aspirating inlet (41), an aspirating outlet (42), and an aspirating channel (43), the sheath (12) of the visualized medical system (100) with a cavity expanding device is a tube with two cavities comprising one cavity provided with the observation mechanism (2) and the other cavity constituting or provided with the aspirating channel (43), the aspirating inlet (41) is arranged at a front end of the sheath (12), and the aspirating outlet (42) of the negative-pressure aspirating mechanism (4) is arranged at a rear end of the sheath (12).

13. The visualized medical system with a cavity expanding device according to claim 11, wherein a negative-pressure control switch (44) configured to control a negative-pressure status is arranged on the negative-pressure aspirating mechanism (4).

14. The visualized medical system with a cavity expanding device according to claim 13, wherein the negative-pressure control switch (44) is arranged on the handle (52) of the operating mechanism (5).

15. The visualized medical system with a cavity expanding device according to claim 1, wherein the visualized medical system (100) with a cavity expanding device further comprises a flushing mechanism (6), and at least one water outlet (61) of the flushing mechanism (6) is located at a front end of the camera (22-1) of the lens module (22).

16. The visualized medical system with a cavity expanding device according to claim 15, wherein the sheath (12) of the visualized medical system (100) with a cavity expanding device is a tube with three tubes comprising a first cavity provided with the observation mechanism (2), a second cavity constituting or provided with the aspirating channel (43), and a third cavity constituting a water inlet pipe (63) of the flushing mechanism (6) or configured for entry and exit of a surgical instrument, and a water inlet (62) of the flushing mechanism (6) is arranged at a rear end of the sheath (12).

17. The visualized medical system with a cavity expanding device according to claim 15, wherein a flushing switch (64) configured to control a flushing water flow is arranged on the flushing mechanism (6).

18. The visualized medical system with a cavity expanding device according to claim 17, wherein the flushing switch (64) is arranged on the handle (52) of the operating mechanism (5).

19. The visualized medical system with a cavity expanding device according to claim 2, wherein the expanding mesh (11-1) of the cavity expanding mechanism (1) is a woven mesh made of a wire.

20. The visualized medical system with a cavity expanding device according to claim 2, wherein the expanding mesh (11-1) has varying geometrical shapes.

21. The visualized medical system with a cavity expanding device according to claim 2, wherein the expanding mesh (11-1) is made of a medical elastic material.

22. The visualized medical system with a cavity expanding device according to claim 21, wherein the expanding mesh (11-1) is made of medical elastic stainless steel or medical titanium-nickel shape memory alloy.

23. The visualized medical system with a cavity expanding device according to claim 21, wherein the expanding mesh (11-1) is made of a medical transparent elastic polymer material.

24. The visualized medical system with a cavity expanding device according to claim 1, wherein a coating is arranged on a protection cover (20) of the observation mechanism (2).

25. The visualized medical system with a cavity expanding device according to claim 1, wherein the observation mechanism (2) comprises at least two cameras (22-1).

26. The visualized medical system with a cavity expanding device according to claim 25, wherein at least one of the cameras (22-1) is arranged at the front end (5-1) of the operating mechanism (5).

27. The visualized medical system with a cavity expanding device according to claim 25, wherein at least one of the cameras (22-1) is arranged on a side surface (5-5) of the operating mechanism (5).

28. The visualized medical system with a cavity expanding device according to claim 25, wherein observation areas of two or more of the cameras (22-1) are contiguous or partially overlapped.

29. The visualized medical system with a cavity expanding device according to claim 1, wherein the observation mechanism (2) further comprises an instrument channel (25).

30. The visualized medical system with a cavity expanding device according to claim 1, wherein an electrical interface (31) connected to a host is arranged on the circuit system (3).

31. The visualized medical system with a cavity expanding device according to claim 1, wherein the visualized medical system (100) with a cavity expanding device comprises an electronic protection mechanism (30), and the electronic protection mechanism (30) is arranged on the outside of the observation module (2) and/or the circuit system (3) and configured to perform waterproof, gasproof, and insulation protection on electronic components of the observation module (2) and/or the circuit system (3).

32. The visualized medical system with a cavity expanding device according to claim 1, wherein the visualized medical system (100) with a cavity expanding device further comprises an identification system (8).

33. The visualized medical system with a cavity expanding device according to claim 1, wherein the visualized medical system (100) with a cavity expanding device further comprises a flushing system (106), a water inlet (106-1) of the flushing system (106) is connected to an infusion bottle or bag (7) by a water pipe (106-4), and a water outlet (106-2) of the flushing system (106) is connected to a water inlet (62) of a flushing mechanism (6) of the visualized medical system (100) with a cavity expanding device by the water pipe (106-4).

34. The visualized medical system with a cavity expanding device according to claim 33, wherein the flushing system (106) is driven by a peristaltic pump (106-3).

35. The visualized medical system with a cavity expanding device according to claim 1, wherein the visualized medical system (100) with a cavity expanding device further comprises a negative-pressure aspirator (105), and an aspirating outlet (42) of a negative-pressure aspirating mechanism (4) of the visualized medical system (100) with a cavity expanding device is connected to the negative-pressure aspirator (105).
